(19) Europäisches Patentamt
European Patent Office
Office européen des brevets

(11) EP 4 223 303 A1

(12) EUROPEAN PATENT APPLICATION
published in accordance with Art. 153(4) EPC

(43) Date of publication:
**09.08.2023 Bulletin 2023/32**

(21) Application number: **21875872.0**

(22) Date of filing: **01.10.2021**

(51) International Patent Classification (IPC):
***A61K 36/17*** *(2006.01)* ***A23L 33/105*** *(2016.01)*
***A61P 31/14*** *(2006.01)*

(52) Cooperative Patent Classification (CPC):
**A23L 33/105; A61K 36/17; A61P 31/14;**
Y02A 50/30

(86) International application number:
**PCT/JP2021/036413**

(87) International publication number:
**WO 2022/071576 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2020 JP 2020167897**

(71) Applicants:
- **The Kitasato Institute**
  **Tokyo 108-8641 (JP)**
- **Japan As Represented By Director General Of National Institute Of Health Sciences**
  **Kawasaki-ku**
  **Kawasaki-shi**
  **Kanagawa 210-9501 (JP)**
- **Matsuyama University**
  **Matsuyama-shi, Ehime 790-8578 (JP)**
- **Tokiwa Phytochemical Co., Ltd.**
  **Sakura-shi, Chiba 285-0801 (JP)**
- **TSUMURA & CO.**
  **Minato-ku**
  **Tokyo 107-8521 (JP)**

(72) Inventors:
- **HANAWA, Toshihiko**
  **Tokyo 108-8641 (JP)**
- **HYUGA, Sumiko**
  **Tokyo 108-8641 (JP)**
- **ODAGUCHI, Hiroshi**
  **Tokyo 108-8641 (JP)**
- **GODA, Yukihiro**
  **Kawasaki-shi, Kanagawa 210-9501 (JP)**
- **HYUGA, Masashi**
  **Kawasaki-shi, Kanagawa 210-9501 (JP)**
- **UEMA, Masashi**
  **Kawasaki-shi, Kanagawa 210-9501 (JP)**
- **ASAKURA, Hiroshi**
  **Kawasaki-shi, Kanagawa 210-9501 (JP)**
- **UCHIYAMA, Nahoko**
  **Kawasaki-shi, Kanagawa 210-9501 (JP)**
- **AMAKURA, Yoshiaki**
  **Matsuyama-shi, Ehime 790-8578 (JP)**
- **YOSHIMURA, Morio**
  **Matsuyama-shi, Ehime 790-8578 (JP)**
- **YANG, Jinwei**
  **Sakura-shi, Chiba 285-0801 (JP)**
- **MIZOGUCHI, Kazushige**
  **Inashiki-gun, Ibaraki 300-1192 (JP)**

(74) Representative: **Hoffmann Eitle**
**Patent- und Rechtsanwälte PartmbB**
**Arabellastraße 30**
**81925 München (DE)**

# (54) ANTIVIRAL AGENT FOR PREVENTING OR TREATING COVID-19 INFECTION

(57) Provided is the use of a pharmaceutical preparation, a Kampo preparation, a food, or another composition comprising an Ephedra Herb extract, an ephedrine alkaloids-free Ephedra Herb extract, or Ephedra Herb macromolecule condensed-tannins as an active ingredient for prevention or treatment of COVID-19. Each composition is preferably provided in an orally ingestible form.



Processed by Luminess, 75001 PARIS (FR)

(Cont. next page)

Fig. 1

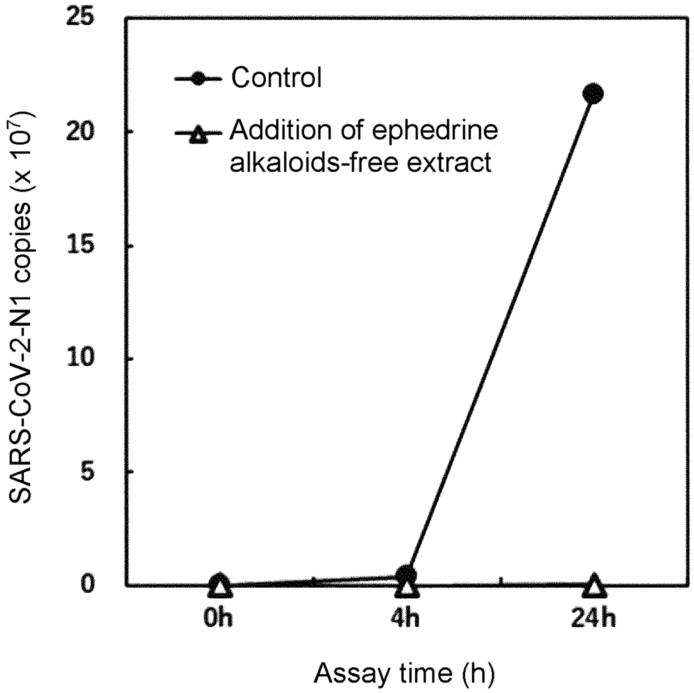
Control
Addition of ephedrine alkaloids-free extract
SARS-CoV-2-N1 copies (x 10^7)
25
20
15
10
5
0
0h
4h
24h
Assay time (h)

**Description**

TECHNICAL FIELD

The present invention relates to an antiviral agent that can be used to prevent or treat COVID-19 and comprises, as an active ingredient, Ephedra Herb extract, ephedrine alkaloids-free Ephedra Herb extract (EFE) obtainable by removing ephedrine alkaloids from Ephedra Herb extract, and/or Ephedra Herb Macromolecule Condensed-Tannins (EMCT).

BACKGROUND ART

The global spread of Coronavirus disease 2019 (COVID-19) has made the establishment of prophylactic and therapeutic protocols an urgent issue. In view of this background, research is being conducted to find effective drugs for treatment of COVID-19 from various existing drugs as soon as possible. However, it is uneasy to predict whether drugs that have been proven effective against viruses other than coronaviruses will be effective against severe acute respiratory syndrome coronavirus 2 (SARS-CoV-2). Efficacy needs to be verified by evaluation experiments at the molecular and cellular levels based on the infection mechanism.

The following table shows the drugs approved in Japan to date (Non-Patent Literature 1: Guide to Medical Treatment of New Coronavirus Infections, Version 5.3, August 29, 2021/Nikkan Yakugyo, September 27, 2021). Except for the antibody drugs Ronapreve and Zebudi, these drugs are for the treatment of patients with moderate symptoms or severer. Ronapreve and Zebudi are intravenous infusions, and there is a limit to the number of medical institutions where treatment can be implemented. The price of each drug is said to be more than 100,000 yen, and it is difficult to increase the volume of production of the antibodies. Thus, there is a need for an oral therapeutic drug that can be safely and inexpensively administered to persons with mild symptoms under home care.

[Table 1]

| Drug | Subi ects |
|---|---|
| Remdesivir | Patients with pneumonia caused by SARS-CoV-2 |
| Dexamethasone | Patients in need of oxygenation |
| Baricitinib | To administer in-hospital patients with moderate II to severe symptoms |
| Ronapreve (casirivimab/imdevimab: antibody medicine) | Patients (with mild to moderate I symptoms) who have risk factors for severe infection due to SARS-CoV-2 and do not require oxygenation |
| Zebudi (antibody medicine) | Patients with "mild to moderate I symptoms" who are at risk for severe symptoms and do not require oxygenation |

Kampo medicines are combined crude drugs and have been named and used as a separate unit for treatment of various diseases. Kampo medicines have been applied and prescribed based on the medical theory of Japanese traditional medicine, Kampo, which is different from modern medicine. Thus, the treatment of COVID-19 with Kampo medicines has been expected. In view of such a background, several Kampo medicines have been proposed as candidate drugs based on the previous findings in addition to the medical theory of Kampo (NPLs 2 and 3). However, there is no concrete evidence, and only its possibilities are presented. In addition, it has been proposed that the Chinese medicine Qingfei Paidu decoction, which is an extract of several herbal medicines traditionally used in China, could be a candidate drug (NPLs 2 and 4). However, the mechanism of action of Qingfei Paidu decoction as an antiviral agent is unknown. It has not been reported whether all herbal medicines, or specific combinations of herbal medicines, or a specific herbal extract alone are/is effective.

As mentioned above, crude drugs are used as traditional medicines such as Kampo medicine. The crude drugs are cultivated on the farm or harvested from the wild, dried, and used. Accordingly, even for the same species, the component content may vary due to uncertain factors such as the season of collection and weather conditions. It is possible to empirically use, for instance, crude drugs or Kampo medicines that exert antiviral actions. However, the titer of a lot from which the samples were collected is indeterminate. Therefore, in order to provide a drug with an efficacy indication, appropriate tests must be conducted and the indication has to be confirmed such that the drug conforms to the appropriate specifications.

Ephedra Herb is a drug listed in the Japanese Pharmacopoeia, and its efficacy has been believed to be almost entirely due to ephedrine alkaloids. However, the present inventors have found that Ephedra Herb exerts useful efficacy independent of ephedrine alkaloids, and have arrived at the idea that an Ephedra Herb extract can be provided as a highly safe drug by selectively removing ephedrine alkaloids, which are substances responsible for adverse effects. Then, it has been indicated that the EFE is useful as an anticancer/anti-metastasis drug, an analgesic, or an anti-influenza viral drug (PTL 1 and NPL 5). Further, the present inventors have revealed that EMCT with a weight average molecular weight of 45,000 or more participates in the above efficacy (PTL 2 and NPL 6).

Thus, an Ephedra Herb extract, EFE, or EMCT has been shown to exert some efficacy. However, there is no report of antiviral action against SARS-CoV-2. In addition, based on the previous findings, it has also been unclear, even to those skilled in the art, whether or not the drug exhibits anti- SARS-CoV-2 effects.

If an Ephedra Herb extract, EFE, or EMCT has been revealed to have anti-SARS-CoV-2 activity, they can be provided as an anti-SARS-CoV-2 agent that can be used to prevent or treat COVID-19. Also, a method of quantifying the main active ingredient should be developed. This can guarantee the antiviral actions of Ephedra Herb used as a component of Kampo medicines and makes it possible to provide, as a drug, not only an Ephedra Herb extract or EFE, but also an Ephedra Herb-containing Kampo medicine that is empirically used for treatment of COVID-19.

CITATION LIST

PATENT LITERATURE

PTL 1: WO2015/076286 (Japanese Patent Application No. 2015-549167)

PTL 2: Japanese Patent Laid-Open No. 2019-131536


NON PATENT LITERATURE

NPL 1: Guide to the Medical Treatment of New Coronavirus Infections, Version 5.3, August 29, 2021.

NPL 2: Watanabe Kenji, Jin Chengiun, Shibayama Chikano, Liu Jianping, Jia Liqun, Jin, Yan Hongrong [Urgent Contribution] The Role of Kampo in New Coronavirus Infection (COVID-19), Japan Medical Journal, No. 5008, p44 (April 18, 2020)

NPL 3: Keiko Ogawa, (Special Contribution) How to Consider Kampo Therapy for COVID-19 Infections (Revised 2nd Edition), The Japanese Association for Infectious Diseases Website, http://www.kansensho.or.jp/modules/news/index.php?content_id=147

NPL 4: Ryutaro Arita, Makoto Takayama, Kota Ishizawa, Tadashi Ishii, Report of Qingfei Paidu decoction for COVID-19 in China, The Japanese Association for Infectious Diseases Website, http://www.kansensho.or.jp/modules/news/index.php?content_id=147

NPL 5: Oshima N, Yamashita T, Hyuga S, Hyuga M, Kamakura H, Yoshimura M, Maruyama T, Hakamatsuka T, Amakura Y, Hanawa T, Goda Y, Efficiently prepared ephedrine alkaloids-free Ephedra Herb extract: a putative marker and antiproliferative effects. J Nat Med. 2016; 70: 554-62.

NPL 6: Yoshimura M, Amakura Y, Hyuga S, Hyuga M, Nakamori S, Maruyama T, Oshima N, Uchiyama N, Yang J, Oka H, Ito H, Kobayashi Y, Odaguchi H, Hakamatsuka H, Hanawa T, Goda Y, Quality Evaluation and Characterization of Fractions with Biological Activities in Ephedra Herb Extract and Ephedrine Alkaloids-Free Ephedra Herb Extract. Chem Pharm Bull. 2020; 68(2): 140-149.


SUMMARY OF INVENTION

TECHNICAL PROBLEM

The present invention addresses the problem of providing an antiviral agent characterized as being used to prevent or treat COVID-19.

SOLUTION TO PROBLEM

In the present invention, the anti-SARS-CoV-2 effects of existing drugs or drug candidate compositions should be verified using evaluation experiments at the molecular and cellular levels based on the mechanism of SARS-CoV-2 infection.

The present inventors evaluated, in experiments at the molecular and cellular levels based on the mechanism of SARS-CoV-2 infection, a useful composition that can be used to prevent or treat COVID-19, such as an Ephedra Herb extract, EFE, or EMCT. As a result, they were found to have anti-SARS-CoV-2 activity. Further, the present inventors have found a method of guaranteeing the efficacy of the composition by using a technique to separate the macromolecule condensed-tannins, which are the main component of the anti-SARS-CoV-2 activity, by gel permeation chromatography (GPC). Then, the invention has been completed.

Specifically, the invention provides the following (1) to (11) as representative items.

(1) An antiviral agent comprising an Ephedra Herb extract as an active ingredient for use in prevention or treatment of CO VID-19.
(2) An antiviral agent comprising, as an active ingredient, an EFE obtainable by eliminating ephedrine alkaloids from an Ephedra Herb extract, for use in prevention or treatment of COVID-19.
(3) An antiviral agent comprising EMCT as an active ingredient for use in prevention or treatment of CO VID-19.
(4) The antiviral agent according to any one of the above items, which is in a form of pharmaceutical preparation or Kampo preparation.
(5) The antiviral agent according to item (2) or (3), which is in a form of food.
(6) A method for preventing SARS-COV-2 infection, comprising having orally ingested a food or beverage comprising EFE or EMCT (desirably, medical practice is excluded).
(7) A method for preventing or treating COVID-19, comprising administering to a subject a medicament comprising, as an active ingredient, an Ephedra Herb extract, an EFE, or EMCT.
(8) A method of guaranteeing an antiviral agent efficacy of a composition according to any one of items (1) to (5), comprising quantifying the macromolecule condensed-tannins by GPC.
(9) The antiviral agent according to item (1), wherein the Ephedra Herb extract comprises the macromolecule condensed tannins in an amount of 0.01% or higher.
(10) The antiviral agent according to item (2), wherein the EFE comprises the macromolecule condensed-tannins in an amount of 0.01% or higher.
(11) The method according to item (6) or (7), further comprising administering to the subject another drug for treating COVID-19.

ADVANTAGEOUS EFFECTS OF INVENTION

The invention in the present application has revealed that the Ephedra Herb extract, EFE, or EMCT can be provided as an anti-SARS-CoV-2 drug. In addition, the main ingredient responsible for the anti-SARS-CoV-2 action has been found to be macromolecule condensed-tannins with molecular weights of 10,000 to 500,000. This made it possible to establish a method of quantifying and guaranteeing antiviral agent efficacy by GPC.

As already mentioned, there is a need for an orally administered drug that is safe and inexpensive and can be administered to patients with COVID-19. The following table shows oral drugs under development worldwide as of September 2021.

[Table 2]

| Status of Oral COVID-19 Drugs under Development Worldwide | | |
|---|---|---|
| Drug | Mechanism of Action | Status of Clinical Trial |
| **EFE** (Kitasato University, National Institute of Health Sciences, TSUMURA & CO.) | The drug can bind to a spike protein of the virus, thereby inhibiting the binding of the virus to a host cell.<br>The drug has the same mechanism of action as of an antibody medicine Ronapreve. | Investigator-initiated clinical trial<br>Phase 1 for COVID-19 patients with mild symptoms is underway.<br>Phase 2 is planned to be implemented from October 2021 to January 2022. |
| Molnupiravir (MK-448) (MSD Pharmaceutical*) *Subsidiary of Merck (Germany) | RNA polymerase inhibitor (an enzyme that catalyzes replication of the viral genome) | Corporate clinical trial<br>Phase 3 (international clinical trials in Japan, the U.S., and Europe) is ongoing.<br>Results will be available around October. |

(continued)

| Status of Oral COVID-19 Drugs under Development Worldwide | | |
|---|---|---|
| Drug | Mechanism of Action | Status of Clinical Trial |
| PF-07321332 (PF-07321332 is administered in combination with an anti-HIV drug Ritonavir) Pfizer Inc. (the U.S.) | Main protease inhibitor (A long protein is translated from the viral RNA, and is cleaved by main protease. Then, each fragment functions as a viral structural protein or enzyme.) | Corporate clinical trial International Phase 2/3 (the U.S., Argentina, India, Korea, Puerto Rico, Taiwan, Turkey, Japan will also participate) initial data will be available in October-December. |
| S-217622 SHIONOGI & Co., Ltd. | Main protease inhibitor | Corporate clinical trial<br>Domestic Phase 1 for healthy subjects is underway. |
| AT-527 Chugai Pharmaceutical (Exclusive rights to develop and market the product in Japan have been obtained from Roche, Ltd.) | RNA polymerase inhibitor | Corporate clinical trial<br>Phase 2 is in progress overseas. |
| Ivermectin (Kitasato University) (Kowa Company, Ltd.) | Main protease inhibitor? | Investigator-initiated clinical trial: Phase 2 is underway<br>A corporate clinical trial will be conducted. |

The drugs other than EFE involve either the RNA polymerase inhibitor or main protease inhibitor. No oral drugs have the same mechanism of action as of EFE. EFE has the same mechanism of action as of Ronapreve or Zebudi, and can bind to a spike protein of SARS-CoV2, thereby inhibiting binding to a host cell. Even if the oral drugs in the table above are commercialized before EFE, they can be used in combination with EFE because their mechanisms of action are different. Moreover, since the oral drugs are taken at home by patients, safety is the most important. Here, EFE is safe because the adverse effect-causing components have been removed. Further, EFE may be supplied more economically than Ronapreve or Zebudi, and can be considered to be able to be prophylactically administered to medical professionals, persons with close contact, and/or those who cannot receive vaccination in view of the mechanism of action. Recently, EFE has been found to be effective against variants of SARS-CoV-2. As demonstrated in working examples, EFE inhibits infection of various variants of SARS-CoV-2. Thus, it is possible to respond to the threat of newly emerging variants.

The active ingredient of EFE is considered to be a mixture of condensed tannins with high-molecular weights (or EMCT), which has various structures and molecular weights. The present inventors are not bound to any theory, but believe that the reason why an Ephedra Herb extract, EFE, or EMCT can exert various efficacy (can be used as an anticancer/anti-metastasis drug, an analgesic, or an anti-influenza viral drug) seems to be explained by such diverse structures and varied molecular weights. The present inventors have verified that EFE or EMCT can exert an effect of inhibiting influenza virus infection. Structural differences between influenza viruses and SARS-CoV-2 are larger than those between SARS-CoV-2 variants. However, since both viruses can be inhibited, EFE or EMCT should be widely applicable to new coronavirus (SARS-CoV-2) variants that will emerge in the future.

In view of the above, it is possible to use, for treatment of COVID-19, an anti-SARS-CoV-2 drug, which is either a medical-use or over-the-counter drug, comprising, as an active ingredient, an Ephedra Herb extract, EFE, and/or EMCT. Accordingly, hospitalization is unnecessary, and the use can prevent patients with mild symptoms who is at initial stages of infection and stay home or under care in residential facilities, from turning severe Covid-19.

In addition, since the anti-SARS-COV-2 drug can be provided as an over-the-counter drug, early prophylactic treatment through self-medication will be possible for medical professionals or persons with close contact. Further, EFE or EMCT are free of ephedrine alkaloids, which are responsible for adverse effects. Thus, they can be used as a food in terms of the food and drug classification. Hence, they can be provided as a health functional food that should prevent COVID-19.

Furthermore, it is possible to provide a method of guaranteeing, as an anti-SARS-COV-2 drug, efficacy of Ephedra Herb-containing Kampo medicines, as well as the Ephedra Herb extract, EFE, and macromolecule condensed-tannins.

BRIEF DESCRIPTION OF DRAWINGS

Fig. 1 is a graph showing effects on viral copy number with or without addition of EFE to SARS-CoV-2-infected cells. The graph shows the differences of N RNA copy number of SARS-CoV-2 in VeroE6/TMPRSS2 cells with (triangles) or without (closed circles) EFE. The x-axis: time elapsed after virus inoculation; the y-axis: SARS CoV-2 RNA copy number.

Fig. 2 shows the inhibitory effect of EFE on infection with variant strains of SARS-CoV-2.

Fig. 3 shows a sensorgram indicating binding of some ingredients of EFE to the S 1 domain of spike protein of SARS-CoV-2, which is involved in the infection. The measured values (solid line) are shown with predicted values (dashed line) obtained by curve-fitting using a binding model.

Fig. 4 shows sensorgrams indicating binding of EMCT to spike protein of SARS-CoV-2, which is involved in the infection. Each measured value (each solid line) for EMCT at 400 nM, 200 nM, or 100 nM is shown with each predicted value (each dashed line) obtained by curve-fitting using a binding model.

Fig. 5 is a graph showing inhibition of spike protein of SARS-CoV-2 binding to ACE2 by EMCT. The four-parameter logistic regression (solid line) showed that the IC50 was calculated to be 3 $\mu$g/mL.

Fig. 6 indicates the levels of virus titer in the right lung on day 5 of MHV infected mice.

Fig. 7 indicates the respiratory status on day 5 of MHV infected mice.

Fig. 8 is diagrams illustrating the structure of proanthocyanidin A-type or proanthocyanidin B-type and the structures of their constituent units.

DESCRIPTION OF EMBODIMENTS

The invention provides use of an Ephedra Herb extract, EFE, or EMCT for prevention or treatment of COVID-19, in an anti-viral agent, and a composition, such as a drug or a food, containing them as an active ingredient.

Preparation of Ephedra Herb Extract

The terrestrial stem of a plant of Ephedraceae such as *Ephedra sinica* Stapf, *Ephedra intermedia* Schrenk et C.A. Meyer, or *Ephedra equisetina* Bunge (Ephedraceae) may be used to prepare an Ephedra Herb extract used in the invention. Fresh, dried, or processed terrestrial stems are available. The process of extracting an Ephedra Herb extract can be performed according to any of well-known procedures. Examples of an extraction solvent used include water, warm water, hot water, an alcohol-based solvent, or another organic solvent such as acetone. Examples of the alcohol-based solvent include methanol (MeOH), ethanol, propanol, isopropanol, butanol, or isobutanol. These solvents may be used singly or in combination.

The amount of extraction solvent should be 2-100 parts by weight, 4-50 parts by weight, or 8-25 parts by weight based on the dry weight of Ephedra Herb. The extraction temperature is preferably from 4 to 98°C. The extraction time is preferably from 30 min to 2 h. The extraction procedure may use any process such as agitation extraction, immersion extraction, countercurrent extraction, ultrasonic extraction, and/or supercritical extraction. In one embodiment, the extraction solvent is water, and the Ephedra Herb extract may be a hot water extracted liquid and/or a hot water extracted material of Ephedra Herb. The hot water extracted liquid and/or hot water extracted material of Ephedra Herb may be prepared by extraction using 2-100, 4-50, or 8-25 parts by weight, or about 10 parts by weight of hot water (e.g., at about 95°C) based on dried Ephedra Herb raw material for 30 min to 2 h.

The resulting extract, a filtrate obtained by filtering the extract, a concentrated liquid obtained by concentrating the filtrate, or a dried material obtained by drying the concentrated liquid may be used as a raw material for producing an EFE or EMCT.

In addition, Ephedra Herb extract may be used as and applied as a bulk Ephedra Herb extract or an extract of raw materials for Kampo medicine in an antiviral agent indicated for prevention or treatment of COVID-19. In this case, it is preferable to contain 0.01% or more of the macromolecule condensed tannins with molecular weights of 10,000 to 500,000 as described below, and even more preferable to contain 0.1% or more of the macromolecule condensed tannins. Such an Ephedra Herb extract shown to contain the macromolecule condensed tannins with molecular weights of 10,000 to 500,000 at 0.01% or more or 0.1% or more is also within the scope of the invention.

Preparation of Ephedrine Alkaloids-Free Ephedra Herb Extract (EFE)

EFE used in the invention can be obtained by using an Ephedra Herb extract as a raw material, removing ephedrine by cation exchange chromatography, and then concentrating and drying the extract.

EFE is a product obtainable by removal of ephedrine alkaloids from an Ephedra Herb extract. The content of ephedrine alkaloids (the total content of ephedrine and pseudoephedrine) is preferably 0.23% or less, more preferably 0.023% or less, and still more preferably 0.05 ppm (detection limit) or less.

The Japanese Pharmacopoeia specifies that the dried Ephedra Herb medicine contains 0.7% or more of total alkaloids (ephedrine and pseudoephedrine). This amount is calculated as the content of about 2.3% to 3.5% or more in terms of total alkaloids in the Ephedra Herb extract. Thus, EFE is clearly distinguished from the Ephedra Herb extract.

Note that the above content in terms of total alkaloids in the Ephedra Herb extract can be calculated with reference to "Kampo No Shucho (Opinion of Kampo)-Modern Sciences and Kampo Preparations" (Ichiro Narikawa, p162-163, Kenyukan, Inc., published on 1991) and Reference Example 1. When an Ephedra Herb extract is prepared from Ephedra Herb according to the Japanese Pharmacopoeia, almost all ephedrine alkaloids contained in the Ephedra Herb are transferred to the Ephedra Herb extract. However, since the yield of the Ephedra Herb extract obtained from the Ephedra Herb is from 20% to 30%, the total alkaloids contained in the Ephedra Herb extract are concentrated 3.3- to 5-fold. Therefore, the standard value for the total alkaloids in an Ephedra Herb extract is from 2.3% to 3.5% or more, which numerical value is 3.3 to 5 times the "0.7% or more", namely the value for the total alkaloids in Ephedra Herb as specified in the Japanese Pharmacopeia. In EFE used in the invention, the content of ephedrine alkaloids should be one-tenth or less of that in the Ephedra Herb extract. This means that the possibility of occurrence of adverse effects due to ephedrine alkaloids would be very small.

For a cation-exchange column suitable for the production of EFE, a packing material can be selected according to ephedrine alkaloids removal efficiency. An EFE with an ephedrine alkaloids content of 0.23% or less may be produced. In this case, the column packing material can be selected from a weakly acidic cation exchange resin WK20 or a strongly acidic cation exchange resin SK104, SK110, SK1B, UBK530, PK216, IR120B, FPC3500, or 1060H. An EFE with an ephedrine alkaloids content of 0.023% or less may be produced. In this case, the column packing material can be selected from a weakly acidic cation exchange resin WK20 or a strongly acidic cation exchange resin SK104, SK110, SK1B, UBK530, PK216, IR120B, or 1060H. Further, an EFE with an ephedrine alkaloids content of 0.05 ppm or less may be produced. In this case, a strongly acidic cation exchange resin PK216 can be selected as the column packing material, as well as a strongly acidic cation exchange resin SK1B or IR120B can also be selected.

In addition, a bulk EFE may be used as and applied as an antiviral agent indicated for prevention or treatment of COVID-19. In this case, it is preferable to contain 0.01% or more of the macromolecule condensed-tannins with molecular weights of 10,000 to 500,000 as described below, and even more preferable to contain 0.1% or more of the macromolecule condensed-tannins. Such EFE shown to contain the macromolecule condensed-tannins with molecular weights of 10,000 to 500,000 at 0.01% or more or 0.1% or more is also within the scope of the invention.

During the production of EFE, the specific chromatography procedure is any of procedures known to those skilled in the art. The drying procedure may be any procedure, such as drying under reduced pressure, lyophilization, or spray-drying. If necessary, an excipient such as dextrin may be included.

## Preparation of Ephedra Herb Macromolecule Condensed-Tannins (EMCT)

The EMCT in the invention are high-molecular mass (macromolecule) condensed tannins derived from Ephedra Herb with molecular weights ranging from 10,000 to 500,000, and are proanthocyanidins in which proanthocyanidin A-type and proanthocyanidin B-type are polymerized. More specifically, the EMCT are mainly proantocyanidin B-type and partly proantocyanidin A-type units, including pyrogallol- and catechol-type flavan 3-ols as extension and terminal units, and the content ratio between the pyrogallol-type and the catechol-type is from 4 to 5 : 1 (Fig. 8). Here, the molecular weights in EMCT are calculated based on GPC-standard polystyrene as the standard substance.

The EMCT in the invention can be obtained from an Ephedra Herb extract or EFE as a raw material, and by using column chromatography such that a mixed solvent of water and alcohol or water and acetone is used, and elution is conducted while the concentration of alcohol or acetone is gradually increased. The solvent is distilled away from eluate fractions containing macromolecule condensed-tannins with molecular weights of 10,000 to 500,000. Then, the resulting material is dried and used as a bulk drug.

Specifically, an Ephedra Herb extract or EFE is dissolved in water, and the resulting solution is divided gradually using a less polar organic solvent to a highly polar organic solvent to produce a water ($H_2O$) extract. The water extract is subjected to column chromatography in which elution is conducted using a mixed solvent of water and an organic solvent selected from MeOH, ethanol, or acetone while the concentration of the organic solvent is gradually increased. By measuring the molecular weight in each fraction obtained, eluates containing macromolecule condensed-tannins with molecular weights of 10,000 to 500,000 can be obtained.

Examples of the less polar organic solvent include n-hexane, diethyl ether, or ethyl acetate. Examples of the highly polar organic solvent include n-butanol, ethanol, MeOH, or water. During chromatography used for fractionation, it is possible to use a styrenedivinylbenzene-based synthetic adsorbent such as Diaion HP-20, or LH-20 etc., or another aromatic synthetic adsorbent. The molecular weight in each fraction may be measured by, for instance, viscometry, GPC, mass spectrometry, or osmometry.

More specifically, a starting material of chromatography described below may be an Ephedra Herb extract or EFE dissolved in water, or a water extract produced by successive partitioning of the Ephedra Herb extract or EFE using ethyl acetate and water-saturated n-butanol. Each extract obtained may be separated using Diaion HP-20 column chromatography in which stepwise elution is conducted with $H_2O$, 20% MeOH, 40% MeOH, and MeOH in this order to collect $H_2O$ fraction, 20% MeOH fraction, 40% MeOH fraction, and MeOH fraction, respectively. The EMCT with molecular weights of 10,000 to 500,000 can be obtained from the 20% MeOH fraction, 40% MeOH fraction, or MeOH fraction.

The EMCT in the invention may be prepared using the 20% MeOH fraction, 40% MeOH fraction, or MeOH fraction singly or using them in any combination. The corresponding fraction contains preferably 10% or more, and more preferably 40% or more, of the above macromolecule condensed-tannins with molecular weights of 10,000 to 500,000.

The EMCT in the invention can be obtained by, as another procedure, subjecting an Ephedra Herb extract or EFE dissolved in water, or the above water extract, to Sephadex LH-20 column chromatography and washing with 50% MeOH and then 80% MeOH, followed by 70% acetone to collect a 70% acetone fraction as the EMCT.

During the production of EMCT, the specific chromatography procedure may be one of procedures known to those skilled in the art. The bulk drug used may be a solution containing EMCT obtained through chromatography or powder obtained by drying the solution by any procedure such as drying under reduced pressure, lyophilization, or spray drying. Further, an excipient such as dextrin may be added, and the mixture is then dried by the above procedure to prepare a bulk drug with improved physical properties as powder.

## Drug and Treatment Protocol

In an embodiment of the invention, an Ephedra Herb extract, EFE, or EMCT can be used as an active ingredient for an anti-SARS-COV-2 drug.

A pharmaceutical preparation comprising, as an active ingredient, an Ephedra Herb extract, EFE, or EMCT can be administered to a subject in need of prevention or treatment of COVID-19 so as to prevent or treat COVID-19. Thus, the subject includes not only patients with mild or severe symptoms, but also those who are suspected to be infected.

The method of administering a pharmaceutical preparation in the invention is not particularly limited, but an orally administrable dosage form is preferred. The pharmaceutical composition in the invention can be prepared in various dosage forms.

Examples for oral administration include, but are not limited to, tablets, capsules, dispersions, granules, rounds, liquids, emulsions, suspensions, solutions, liquors, syrups, extracts, or elixirs. Various pharmaceutically acceptable carriers can also be added to the preparation.

Examples include, but are not limited to, excipients, binders, disintegrants, lubricants, flavoring agents, colorants, sweeteners, taste masking agents, dissolution aids, suspending agents, emulsifiers, coating agents, vitamin C, and/or antioxidants.

Ephedra Herb is widely used as a constituent herbal medicine in Kampo medicine. Thus, an Ephedra Herb-containing Kampo medicine or a Kampo medicine in which Ephedra Herb is replaced by EFE can be provided as an anti-SARS-COV-2 drug.

## Foods

EFE or EMCT in the invention does not contain ephedrine alkaloids, which are responsible for adverse effects. Thus, they can be used as a food. EFE or EMCT in the invention can be included in the foods. For example, EFE may be blended with raw materials to prepare various food forms. Each food is not particularly limited, and can be selected depending on the purpose. Examples include beverages such as soft drinks, carbonated drinks, nutritional drinks, fruit drinks, and lactic acid drinks; snacks such as sweets, candies, chewables, jellies, gums, and chocolates; and health foods, functional foods, and dietary supplements in various forms such as granules, tablets, capsules, and drinks.

The foods can be general foods, health supplements, nutritional supplements, etc. (what is called supplements) without any specific function indication, or foods with a function indication (e.g., functional health foods). Each food may be a food product with an indication of suitability for a special use (what is so-called a health claim), but does not include a food product to be ingested as a medicine. For example, the health claim could be an indication that the product reduces the risk of a particular disease (e.g., the risk of contracting COVID-19).

Such a food may be admixed with, in addition to EFE or EMCT, inorganic ingredients such as calcium, various vitamins, oligosaccharides, dietary fibers such as chitosan, proteins such as a soybean extract, fats such as lecithin, and/or sugars such as sucrose and lactose.

Making a subject orally ingest a food or beverage comprising EFE or EMCT in order to prevent COVID-19 is also an embodiment of the invention. It seems to be desirable that such an act is implemented as non-medical practice.

Quantitative evaluation of Ephedra Herb Macromolecule Condensed-Tannins (EMCT)

The invention also encompasses quantification of the content of EMCT with a specific molecular weight range. For example, regarding a raw material herbal medicine Ephedra Herb, a bulk Ephedra Herb extract, bulk EFE, or bulk EMCT, the macromolecule condensed-tannins can be an indicator component for anti-SARS-COV-2 activity.

Specifically, the content of the macromolecule condensed-tannins in a composition may be quantified, not only in the above-described pharmaceutical raw material or bulk drug, but also a food etc., to check whether the content specifications are met. Such a quantification test should be set as a quality evaluation method, and this can guarantee the effectiveness of the composition as an anti-SARS-COV-2 drug. Further, similar specifications and test methods may be established for Ephedra Herb-containing Kampo medicines such as Maoto and Kakkonto, to guarantee the effectiveness of such a composition as an anti- SARS-COV-2 drug.

The macromolecule condensed-tannins may be quantified by GPC. The specific procedure is exemplified by, but not limited to, the procedure described in Example 7. For example, from the peak areas $A_T$ and As of a sample solution and an EMCT standard solution respectively, the amount (mg) of macromolecule condensed-tannins in 1 mg of the sample can be calculated.

As described above, the GPC analysis can be used in an acceptance test for Ephedra Herb as a raw material herbal medicine or a procedure for testing active ingredient content specifications of various kinds of bulk drugs or foods.

The macromolecule condensed-tannins can be quantified using a measurement sample in liquid, as it is, or a solution prepared using any solvent in the case of a solid. However, the composition may have a small content of macromolecule condensed-tannins or may contain massive quantities of impurities. In this case, a method of preparing EMCT as described herein, i.e., the extraction procedure or the column chromatography, for instance, should be used as a pretreatment procedure to allow the quantification. In some cases, the sensitivity, accuracy, linearity, etc., of the quantification can be improved as well.

In an embodiment, an Ephedra Herb extract or EFE is prepared, and in the resulting Ephedra Herb extract or EFE, the macromolecule condensed-tannins with molecular weights of 10,000 to 500,000 are quantified. Then, the Ephedra Herb extract or EFE having a content of the macromolecule condensed-tannins of 0.01% or more is used as a bulk drug for an anti-SARS-CoV-2 drug.

Incorporation by Reference

The matters disclosed in NPL 5 (Oshima N, Yamashita T, Hyuga S, Hyuga M, Kamakura H, Yoshimura M, Maruyama T, Hakamatsuka T, Amakura Y, Hanawa T, Goda Y, Efficiently prepared ephedrine alkaloids-free Ephedra Herb extract: a putative marker and antiproliferative effects. J Nat Med. 2016; 70: 554-62) and NPL 6 (Yoshimura M, Amakura Y, Hyuga S, Hyuga M, Nakamori S, Maruyama T, Oshima N, Uchiyama N, Yang J, Oka H, Ito H, Kobayashi Y, Odaguchi H, Hakamatsuka H, Hanawa T, Goda Y, Quality Evaluation and Characterization of Fractions with Biological Activities in Ephedra Herb Extract and Ephedrine Alkaloids-Free Ephedra Herb Extract. Chem Pharm Bull. 2020; 68(2): 140-149) are herein incorporated by reference.

Hereinbelow, the invention will be described in more detail with reference to Examples. However, the invention is not limited to them.

EXAMPLES

[Example 1]

Example 1: Inhibitory Effect of EFE on SARS-CoV-2 Infection

It was examined whether an EFE had an inhibitory effect on, for example, proliferation of SARS-CoV-2 in VeroE6/TMPRSS2 cells, i.e., SARS-CoV-2 susceptible cells.

After the examination to confirm the toxic concentration of EFE to VeroE6/TMPRSS2 cells, the concentration of EFE added was set to 50 μg/mL. As a control, the control group, in which no EFE was added, was examined at the same time.

The growth of SARS-CoV-2 was evaluated as follows. After SARS-CoV-2 infection, VeroE6/TMPRSS2 cells were cultured for a certain period, RNA was extracted and quantified the level of viral N RNA by PCR; and the copy number of N RNA was determined from a standard curve from a standard substance.

VeroE6/TMPRSS2 cells were suspended in 10% FBS-DMEM at $5 \times 10^5$ cells/mL, seeded in three 48-well plates at 200 μL per well, and cultured in a $CO_2$ incubator for 24h. The EFE addition group was provided such that the medium was replaced with serum-free DMEM supplemented with 50 μg/mL EFE; and the control group was provided such that the medium was replaced with serum-free DMEM. Each group was incubated in a $CO_2$ incubator for 1 h. The virus (SARS-CoV-2, JPN/TY/WK-521 strain) was inoculated in each well at MOI (Multiplicity of Infection) = 0.03, and incubated in the $CO_2$ incubator for 1h. The cells were then washed twice with serum-free DMEM, and serum-free DMEM was added. One of the plates was collected as it was (0h), the other two plates were incubated in the $CO_2$ incubator for 4h or 24h after the virus inoculation. The whole cells including the medium in each well were used to extract RNA. Then, RT-qPCR was conducted using SARS-CoV-2-N1 set of primers and probe for detection of SARS-CoV-2 N gene provided from CDC in the U.S. A standard curve was drawn using an N-gene plasmid control provided from CDC in the U.S., and the viral N RNA copy number was determined (Table A).

[Table 3]

Table A

| | SARS-CoV-2 -N1 copies | |
|---|---|---|
| | Control | Ephedrine alkaloids-free Ephedra Herb extract added (50 μg/mL) |
| 0h | 10, 876 | 2, 114 |
| 4h | 4, 033, 113 | 49, 678 |
| 24h | 216, 168, 568 | 363, 091 |

As a result, in the control group without any EFE, approximately 10,000 copies of SARS-CoV-2 (WK-521 strain) N RNA were detected at the start of the assay (0 h), and the viral RNA increased approximately 400-fold at 4 h and 20,000-fold at 24 h. By contrast, in the EFE addition group, approximately 2,000 copies of SARS-CoV-2 (WK-521 strain) N RNA were detected at the start of the assay (0 h), and the RNA increased approximately 25-fold at 4 h and 130-fold at 24 h (Fig. 1).

The viral N RNA copy number of SARS-CoV-2 (WK-521 strain) at the start of the assay was reduced to 20% by the addition of EFE. This has demonstrated that the coronavirus infection at the initial stage is suppressed. The virus proliferation rate was examined over time, and was found to be markedly suppressed by the addition of EFE. The viral N RNA copy number after 24 h was 0.17% of the copy number for the control, and was kept at an extremely low level. These results have revealed that the EFE elicits an inhibitory effect on viral replication in the early and infectious stages of SARS-CoV-2 infection.

[Example 2]

Example 2: Inhibitory Effect on Variant strains infection

Since the outbreak in Wuhan in 2019, variants strains have been reported in many countries. The WHO has designated the Alpha, Beta, and Gamma strains from the United Kingdom, South Africa, and Brazil as variants of particular concern for the spread of infection, etc. These variants have entered Japan since 2020, replacing the originally widespread WK-521 relative strain. Thus, it was examined whether an EFE had an inhibitory effect on, for example, proliferation of each variant strains in VeroE6/TMPRSS2 cells, i.e., SARS-CoV-2 susceptible cells. Table B shows the Japanese domestic isolates of new coronaviruses used in Examples 1 and 2.

[Table 4]

| Table B Domestic isolates of new coronaviruses (distributed by the National Institute of Infectious Diseases) | | | | |
|---|---|---|---|---|
| | | Strain name | Name in the graph | Designation in WHO |
| Virus | 1 | TY/WK-521 | WK-521 | |
| | 2 | QK002 | UK1 | Alpha |
| | 3 | QHN001 | UK2 | |
| | 4 | QHN002 | UK3 | |
| | 5 | TY7-501 | Brazil 1 | Gamma |
| | 6 | TY7-503 | Brazil 2 | |
| | 7 | TY8-612 | South Africa | Beta |

After the examination to confirm the toxic concentration of EFE to VeroE6/TMPRSS2 cells, the concentration of EFE added was set to 50 μg/mL. As a control, the control group, in which no EFE was added, was examined at the same time.

The growth of SARS-CoV-2 variant strains was evaluated as follows. After SARS-CoV-2 infection, VeroE6/TMPRSS2 cells were cultured for a certain period, then RNA was extracted and quantified the level of viral N RNA by PCR; and the copy number of N RNA was determined from a standard curve from a standard substance. Furthermore, the titer of infectious virus in the post-24-h culture supernatant was determined.

VeroE6/TMPRSS2 cells were suspended in 10% FBS-DMEM at $2 \times 10^4$ cells/well, seeded in a 96-well plate, and cultured in a $CO_2$ incubator for 24 h. The EFE addition group was provided such that the medium was replaced with serum-free DMEM supplemented with 50 μg/mL EFE; and the control group was provided such that the medium was replaced with serum-free DMEM. Each group was incubated in a $CO_2$ incubator for 3 h. Each SARS-CoV-2 variant was inoculated in each well at MOI (Multiplicity of Infection) = 0.03, and incubated in the $CO_2$ incubator for 2 h. The cells were then washed once with serum-free DMEM, and serum-free DMEM was added. The whole cells including the medium at 2 h (input) or 24 h after the virus infection were used to extract RNA. Then, RT-qPCR was conducted using SARS-CoV-2-N1 set of primers and probe provided from CDC in the U.S. A standard curve was drawn using an N-gene control plasmid provided from CDC in the U.S., and the viral N RNA copy number was determined.

The titer of infectious virus after 24 h post infection was calculated such that 25 μL of culture supernatant was collected; a 10-fold serial dilution series was prepared; the VeroE6/TMPRSS2 cells, which had been seeded in a 96-well plate, were infected therewith and cultured for 4 days; the cytopathic effect (CPE) was checked under a microscope to determine the number of wells with the CPE; and this number and the dilution factor were used for the calculation.

The results showed that in the control group without any EFE, the average copy number of viral N RNA after 24-h culturing was $2.3 \times 10^7$ copies/well. In the case of the EFE addition group, the average copy number of viral N RNA was $5.8 \times 10^5$ copies/well. The number was about 2.5% of the control group, indicating marked suppression of the RNA proliferation (Fig. 2: the N-gene copy number of each variant at 24 h).

In the case of the control group, the average titer of the infectious virus in culture supernatant after 24-h post infection was $4.9 \times 10^5$ $TCID_{50}$/mL. By contrast, in the case of the EFE addition group, the average titer was $1.8 \times 10^4$ $TCID_{50}$/mL. The average titer for the addition group was about 3.7% of the control group, which was set to 100, indicating marked suppression of the production of infectious virus. These results have revealed that the EFE elicits an inhibitory effect on viral replication of SARS-CoV-2 variant strains.

[Example 3]

Example 3: Ability of EFE Component to Bind to Spike Protein Involving SARS-CoV-2 Infection

When SARS-CoV-2binds to a target cell, the viral spike protein binds to a receptor molecule on the target cell. If any component of EFE binds to the spike protein of SARS-CoV-2, binding between the virus and the target cell is inhibited. Here, the ability of EFE component to bind to the spike protein of SARS-CoV-2 was examined using a molecular interaction analyzer.

The molecular interaction analyzer used was a BIACORE (GE Healthcare). The S1 domain of SARS-CoV-2 spike protein (recombinant S1), produced by genetic recombination, was immobilized (about 500 RU) on a sensor chip CM5 by amine coupling. EFE was dissolved in HEPES buffer (0.01 M HEPES pH 7.4, 0.15 M NaCl, 3 mM EDTA, 0.005% (v/v) SurfactantP20) and adjusted to 200 μg/mL. The sample solution was applied at a flow rate of 30 μL/min for 60 s, and then washed with HEPES buffer for 120 s. A sensorgram was obtained by subtracting the response of the recombinant S1-free flow cell from the response of the flow cell with immobilized recombinant S1. The results showed that some ingredients of EFE was strongly bound to the recombinant S1 (Fig. 3).

These results suggested that SARS-CoV-2 infection is inhibited by some ingredients of Ephedra Herb extract or EFE as they strongly binds to the S1 domain of the spike protein, which plays a key role in SARS-CoV-2 infection.

[Example 4]

Example 4: Binding of EMCT to Spike Protein Involving SARS-CoV-2 Infection.

A molecular interaction analyzer was used to examine whether EMCT, which are considered to be an active ingredient of Ephedra Herb extract or EFE, were able to bind to the spike protein of SARS-CoV-2.

The molecular interaction analysis was performed using the analyzer and the sensor chip described in Example 3 under similar conditions. The EMCT was dissolved in HEPES buffer and adjusted to 40 μg/mL, 20 μg/mL, or 10 μg/mL, respectively. The sample solution was applied at a flow rate of 30 μL/min for 60 s, and then washed with HEPES buffer for 120 s. Sensorgrams were obtained by subtracting the response of the recombinant S1-free flow cell from the response of the flow cell with immobilized recombinant S1. The results revealed that EMCT bind to the recombinant S1 in a concentration-dependent manner (Fig. 4). The average molecular weight of EMCT was about 100,000. Accordingly, the concentration of each sample was determined as 400 nM, 200 nM, or 100 nM. Binding kinetics of EMCT to recombinant S1 was analyzed by curve-fitting method, resulting that the dissociation constant (KD) was calculated to be 30.7 nM. These results predicted that EMCT have sufficient binding ability to function as an inhibitor for binding to the spike protein of SARS-CoV-2.

These results revealed that EMCT, a component of Ephedra Herb extract or EFE, strongly binds to the S 1 domain of the spike protein, which plays a key role in SARS-CoV-2 infection, and suggested that the same as Ephedra Herb extract or EFE, EMCT could be an antiviral agent that inhibits SARS-CoV-2infection.

[Example 5]

Example 5: Inhibitory Effect on Binding between Spike Protein of SARS-CoV-2 and ACE2

It has been revealed that binding of the spike protein of SARS-CoV-2 to ACE2 of a host cell is the first step of infection. As demonstrated in Example 4, EMCT, an active ingredient of Ephedra Herb extract or EFE, can bind to the spike protein. Thus, the enzymatically labeled spike protein was used to examine whether the binding to ACE2 was inhibited by EMCT.

A recombinant ACE2 extracellular domain (binding domain to the spike protein) was immobilized onto a 96-well microplate. Biotinylated recombinant spike protein receptor binding domain (S1-RBD) was added, and the microplate was washed with phosphate buffer. Then, the binding levels of the spike protein was measured with avidin conjugated-HRP and an HRP substrate. EMCT was added at the final concentration of 20 μg/mL, 10 μg/mL, 5 μg/mL, 2.5 μg/mL, or 1.25 μg/mL with biotinylated recombinant S1-RBD to the assay system. Then, the binding levels of the spike protein was measured. The results revealed that EMCT decreases the binding level of the recombinant S 1-RBD against ACE2 in a concentration-dependent manner (Fig. 5). The four-parameter logistic regression showed that the 50% binding inhibitory concentration ($IC_{50}$) was calculated to be 3 μg/mL. These results demonstrated that EMCT inhibits the binding of the spike protein of SARS-CoV-2 to ACE2.

Taken together, these results have revealed that EMCT, which is a component of Ephedra Herb extract or EFE, can inhibit the first stage of SARS-CoV-2 infection, and expected that the same as Ephedra Herb extract or EFE, EMCT could be an antiviral agent that inhibits SARS-CoV-2 infection.

[Example 6]

Example 6: Effect of orally administered EFE on Mouse Coronavirus Infection Model

The *in vivo* effects of EFE were evaluated using mice intranasally infected with murine hepatitis virus (MHV), which belongs to the family Coronaviridae of the order Nidovirales. MHV has been reported to cause pneumonia in mice (Albuquerque, N., et al., J Virol., 80, 10382-10394, 2006).

Mice (BALB/c Cr Slc, female) were inoculated intranasally with MHV (MHV-1/ATCC VR-261), and EFE was administered orally for 6 days, starting 1 h after virus inoculation. The uninfected and infected control groups orally administered water for injection. EFE was administered to the infected groups at 700 mg/kg twice daily [EFE 700×2 (1400)], 700 mg/kg once daily (EFE 700), and 350 mg/kg twice daily [EFE 350×2 (700)]. Ten mice were used in each group. On day 5 after virus inoculation, respiratory status was observed, and the right lung and liver was sampled to measure the viral titer by plaque assay. Animal experiments were approved by the animal care and use committees of Tsumura and Bio Research Center in Japan, and were conducted in accordance with the Basic Guidelines for Conduct of Animal Experiments at Research Institutes under the jurisdiction of the Ministry of Health, Labour and Welfare.

On day 5 after MHV inoculation, the viral titer in the right lung and liver extract was significantly reduced in all the EFE-treated groups compared to the infected control group. The number of viral plaques in the right lung was 1622.3 ± 271.9 (× $10^2$ PFU/g) in the infected control group, 96.1 ± 10.7 in the EFE 700×2 (1400) group, 250.1 ± 67.9 in the EFE 700 group, and 417.5 ± 121.5 in the EFE 350×2 (700) group (Fig. 6). In addition, the respiratory irregularities observed in the infected control group tended to be alleviated in all the EFE-treated groups (Fig. 7).

These results have indicated that orally administered EFE exerts efficacy in the lung.

[Example 7]

Example 7: How to Quantify EMCT

In the method of quantifying EMCT by using GPC shown in Reference Example 8, a quantification method was established in which the column length was doubled to improve impurities separation performance. Using this quantification method, the content of EMCT in each of three lots of Ephedra Herb extract was calculated to be from 0.03 to 0.69% (average 0.36%). In addition, the content of EMCT in one lot of EFE was calculated to be 0.49%.

Quantification Method

Each of a sample or a standard substance for EMCT was weighed and dissolved in a mobile phase to a concentration of about 10.0 mg/mL to prepare each sample solution or a standard substance solution for EMCT. The sample solution or the standard substance solution for EMCT was subjected to GPC under conditions below. Data processing software LabSolutions GPC was used to measure peak areas $A_T$ and As of the sample or the standard substance for EMCT.

GPC Conditions

Column: TSK-gel Super AW4000 (6.0 I.D. × 150 mm × 2 columns) (Tosoh)
Mobile phase: 99.5% *N,N*-dimethylformamide, 0.5% 3 M aqueous ammonium formate
Column temperature: 30°C
Flow rate: 0.3 mL/min
Measurement wavelength: 280 nm
Peak area measurement range: 12-18 min

Calculation Formula

$$\text{Amount (mg) of EMCT per mg sample} = A_T/A_S \times C_S/C_T.$$

$A_T$: Peak area of sample
$A_S$: Peak area of standard substance
$C_T$: Sample concentration (mg/mL)
Cs: Concentration of standard substance for EMCT (mg/mL)

Reference Examples

As already mentioned, the present inventors have previously established a process for producing an EFE with ephedrine alkaloids-independent pharmacological effects such as pain suppression effects by selectively eliminating ephedrine alkaloids from an Ephedra Herb extract, (PTL 1 and NPL 5). The present inventors also established a method for producing EMCT, a new active ingredient obtained from Ephedra Herb extract or EFE as a raw material (PTL 2 and NPL 6). These matters are illustrated by the following Reference Examples. For detailed test conditions and specific data, the descriptions of Examples in PTL 1 (WO2015/076286) and PTL 2 (No. 2019-131536) are incorporated as references.

Reference Example 1: Preparation of Ephedra Herb Extract

Dried Ephedra Herb raw material was pulverized with a mixer, 50 g of the pulverized material was added to 500 mL of water, and the mixture was extracted with stirring at 95°C for 1 h. After solid-liquid separation, the extract was centrifuged at 3000 rpm for 10 min. The resulting supernatant was concentrated under reduced pressure at 60°C and dried under reduced pressure at 60°C overnight to give 9.6 g of Ephedra Herb extract.

Reference Example 2: Examination of Packing Material for Ion Exchange Chromatography

A study was conducted to determine an ion exchange chromatography packing material suitable for the production of EFE. After an Ephedra Herb extract was treated with various ion exchange resins, ephedrine alkaloids contained in the extract were analyzed by TLC and HPLC. A total of 22 types of ion exchange resins were examined: 13 cation exchange resins, 1 amphoteric ion exchange resin, and 8 anion exchange resins. As a result, it was found that the ion-effect resin suitable for ephedrine alkaloid removal is a cation exchange resin. Thus, the content of ephedrine alkaloids contained in the post-treatment extract was quantified by HPLC using a weakly acidic cation exchange resin WK10, WK11, WK20, WK40L, or FPC3500, or a strongly acidic cation exchange resin SK104, SK110, SK1B, UBK530, UBK12, PK216, IR120B, or 1060H. The following table shows the results.

[Table 5]

| Concentration (%) | WK10 | WK11 | WK20 | WK40L | SK104 | SK110 | SK1B | UBK530 | UBK12 | PK216 | IR120B | FPC3500 | 1060H |
|---|---|---|---|---|---|---|---|---|---|---|---|---|---|
| Ephedrine content | 0.16269 | 0.27753 | 0.01595 | 0.22968 | 0.06061 | 0.00638 | 0.01595 | 0.01276 | 0.00319 | 0 | 0.00319 | 0.1276 | 0.00319 |
| Pseudoephedrine content | 0.0682 | 0.1116 | 0.00775 | 0.09765 | 0.0093 | 0 | 0.0031 | 0.00465 | 0 | 0 | 0 | 0.0186 | 0 |
| Ephedrine alkaloid content | 0.23089 | 0.38913 | 0.0237 | 0.32733 | 0.06991 | 0.00638 | 0.01905 | 0.01741 | 0.00319 | 0 | 0.00319 | 0.1462 | 0.00319 |

Reference Example 3: Preparation of EFE by Using Ion Exchange Resin SK1B or IR120B

Dried Ephedra Herb raw material was pulverized with a mixer, 50 g of the pulverized material was added to 500 mL of water, and the mixture was extracted with stirring at 95°C for 1 h. After solid-liquid separation, the extract was centrifuged at 3000 rpm for 10 min. The resulting supernatant was made to pass through 25 mL of strongly acidic cation exchange resin SK1B (manufactured by Mitsubishi Chemical) or strongly acidic cation exchange resin IR120B (manufactured by ORGANO CORPORATION). The filtrate was adjusted to pH = 5.2 with 5% NaHCOs, concentrated under reduced pressure at 60°C, and dried under reduced pressure at 60°C overnight to give 6.3 g of EFE.

Reference Example 4: Comparison of Ephedrine Content between Ephedra Herb extract (Reference Example 1) and EFE (Reference Example 3) by High-Performance Liquid Chromatography

The following table shows the results obtained from HPLC analysis of Ephedra Herb extract (Reference Example 1) or EFE (Reference Example 3). These results have demonstrated that ephedrine alkaloids (ephedrine and pseudoephedrine) can be eliminated from the Ephedra Herb extract to below the detection limit (0.05 ppm) by column chromatography using a strongly acidic cation exchange resin SK1B or IR120B.

[Table 6]

| | Ephedra Herb extract | Ephedrine alkaloids-free Ephedra Herb extract | |
|---|---|---|---|
| | | SK 1 B | IR 1 2 0 B |
| Ephedrine | 3. 19 % | 0.05 ppm or lower | 0.05 ppm or lower |
| Pseudoephedrine | 1. 55 % | 0.05 ppm or lower | 0.05 ppm or lower |

Reference Example 5: Comparison of Compositional Components between Ephedra Herb Extract (Reference Example 1) and EFE (Reference Example 3)

Compositional components were compared by three-dimensional high-performance liquid chromatography (3D-HPLC) between an Ephedra Herb extract and an EFE. As a result, the peaks of ephedrine alkaloids disappeared in the EFE, but the pattern of other components was almost the same as that of the Ephedra Herb extract (data not shown).

In addition, compositional components were compared by LC/MS between the Ephedra Herb extract and the EFE. The results showed that the 1-ephedrine, pseudoephedrine, methylephedrine, and norephedrine peaks disappeared in the EFE (data not shown).

Reference Example 6: Preparation of Ephedra Herb-derived Macromolecule Condensed-Tannins (EMCT)

An EFE (1.0354 g) was added to a Sephadex LH-20 column (1.1 cm × 40 cm, Cytiva) and eluted with 50% MeOH, followed by 80% MeOH, then 70% acetone. The 70% acetone eluate was lyophilized to yield EMCT (125.5 mg).

Reference Example 7: Preparation of Standard Substance for the Macromolecule Condensed-Tannins (EMCT)

About 100 mg of EMCT were weighed precisely and 10 mL of 50% dimethylformamide solution was added accurately, and the mixture was completely dissolved. The resulting solution was analyzed by GPC. As a result, the weight average molecular weight was 85642, and it was verified that there was no peak in the low molecular weight range. The resulting solution was used as a standard substance solution for EMCT (10.0 mg/mL).

Reference Example 8: Evaluation of Ephedra Herb-derived Macromolecule Condensed-Tannins (EMCT) by GPC

Three lots of Ephedra Herb extract were tested by GPC under conditions below. Then, the content of EMCT was calculated to be 2.84 mg/mL.

GPC Test Procedure

Each sample was weighed precisely and dissolved in 50% dimethylformamide solution to prepare a sample solution at a concentration of approximately 10.0 mg/mL. The sample solution or the standard substance solution for EMCT was subjected to GPC under conditions below. Data processing software Chromato-PRO-GPC was used to calculate the peak areas $A_T$ and As of the sample solution or the standard substance solution for EMCT, as well as the weight average molecular weight.

GPC Conditions

Column: TSK-gel Super AW4000 (6.0 I.D. × 150 mm) (Tosoh)
Mobile phase: 99.5% *N,N*-dimethylformamide, 0.5% 3 M aqueous ammonium formate
Column temperature: 30°C
Flow rate: 0.3 mL/min
Measurement wavelength: 280 nm
Peak area measurement range: 5-10 min

$$\text{Amount (mg) of EMCT per mL sample solution} = A_T/A_S \times 10.00.$$

$A_T$: Peak area of sample
$A_S$: Peak area of standard substance
10.00: Amount (mg) of EMCT in 1 mL of standard substance solution

**Claims**

1. An antiviral agent comprising an Ephedra Herb extract as an active ingredient for use in prevention or treatment of COVID-19.

2. An antiviral agent comprising an ephedrine alkaloids-free Ephedra Herb extract (EFE) as an active ingredient for use in prevention or treatment of COVID-19.

3. An antiviral agent comprising Ephedra Herb macromolecule condensed-tannins (EMCT) as an active ingredient for use in prevention or treatment of COVID-19.

**4.** The antiviral agent according to any one of claims 1 to 3, which is in a form of pharmaceutical preparation or Kampo preparation.

**5.** The antiviral agent according to claim 2 or 3, which is in a form of food.

**6.** A method for preventing COVID-19, comprising having orally ingested a food or beverage comprising EFE or EMCT.

**7.** A method for preventing or treating COVID-19, comprising administering to a subject a medicament containing, as an active ingredient, an Ephedra Herb extract, EFE, or EMCT.

**8.** A method of guaranteeing efficacy of an antiviral action of the antiviral agent according to any one of claims 1 to 5, comprising quantifying the macromolecule condensed-tannins by gel permeation chromatography (GPC).

**9.** The antiviral agent according to claim 1, wherein the Ephedra Herb extract comprises the macromolecule condensed-tannins in an amount of 0.01% or higher.

**10.** The antiviral agent according to claim 2, wherein the EFE comprises the macromolecule condensed-tannins in an amount of 0.01% or higher.

**11.** The method according to claim 6 or 7, further comprising administering to the subject another drug for treating COVID-19.

Fig. 1

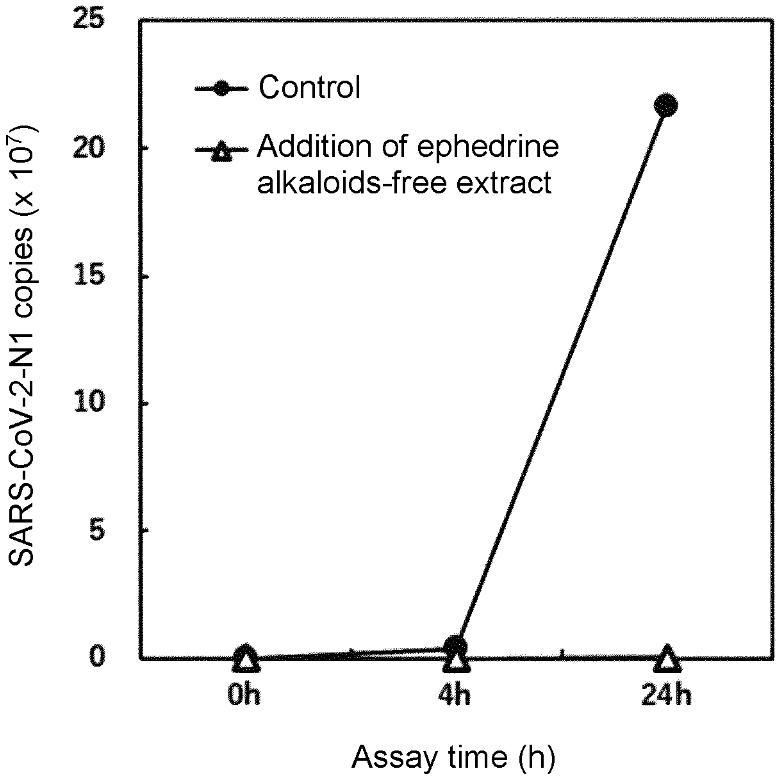
25
20
15
10
5
0
SARS-CoV-2-N1 copies (x 10^7)
Control
Addition of ephedrine alkaloids-free extract
0h
4h
24h
Assay time (h)

Fig. 2

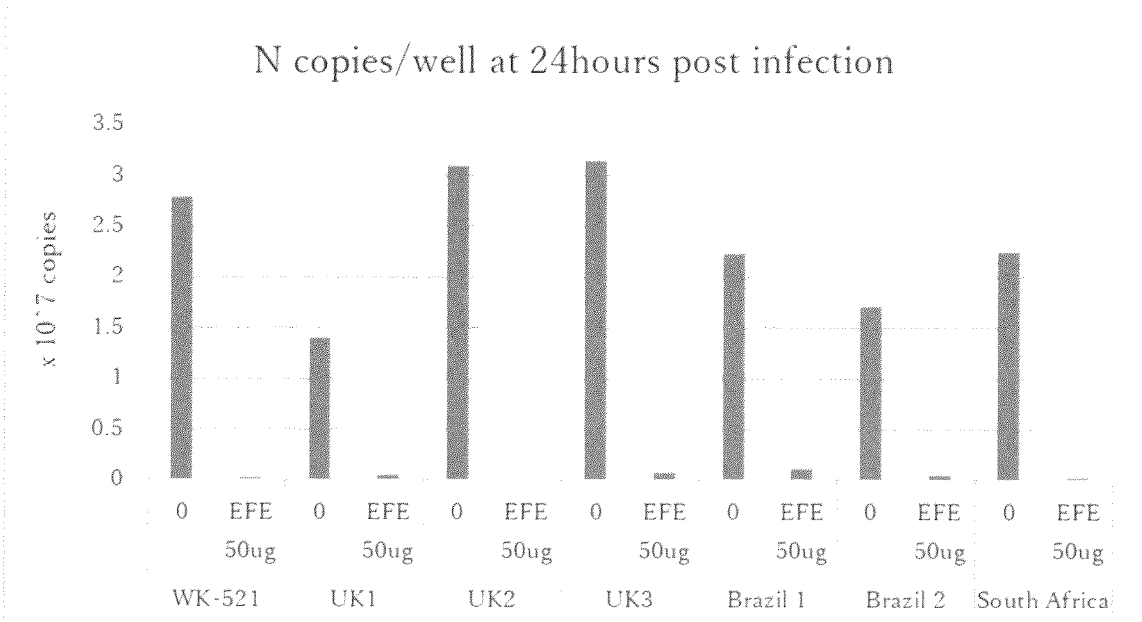
N copies/well at 24hours post infection
x 10^7 copies
3.5
3
2.5
2
1.5
1
0.5
0
0
EFE 50ug
WK-521
UK1
UK2
UK3
Brazil 1
Brazil 2
South Africa

Fig. 3

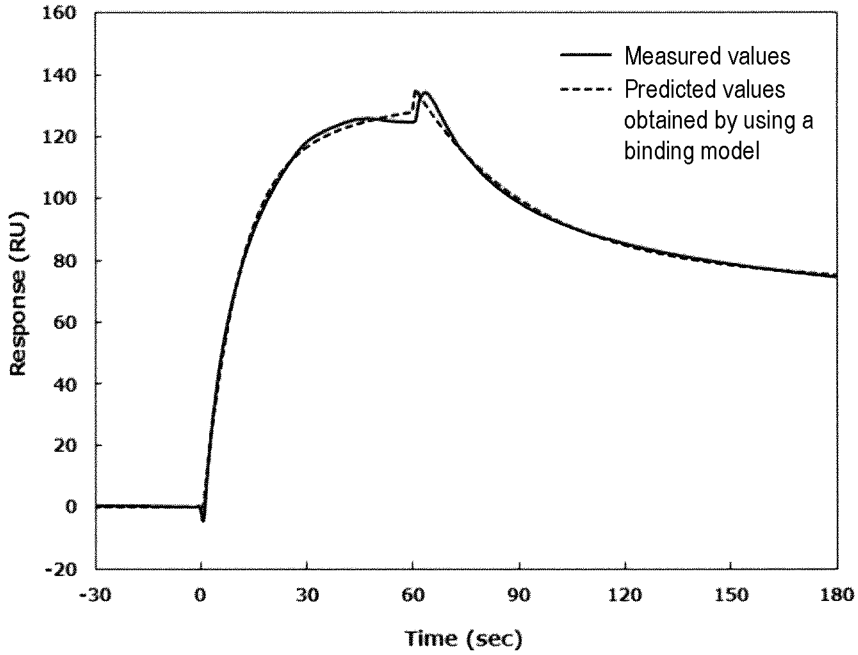
160
140
120
100
80
60
40
20
0
-20
Response (RU)
-30
0
30
60
90
120
150
180
Time (sec)
Measured values
Predicted values obtained by using a binding model

Fig. 4

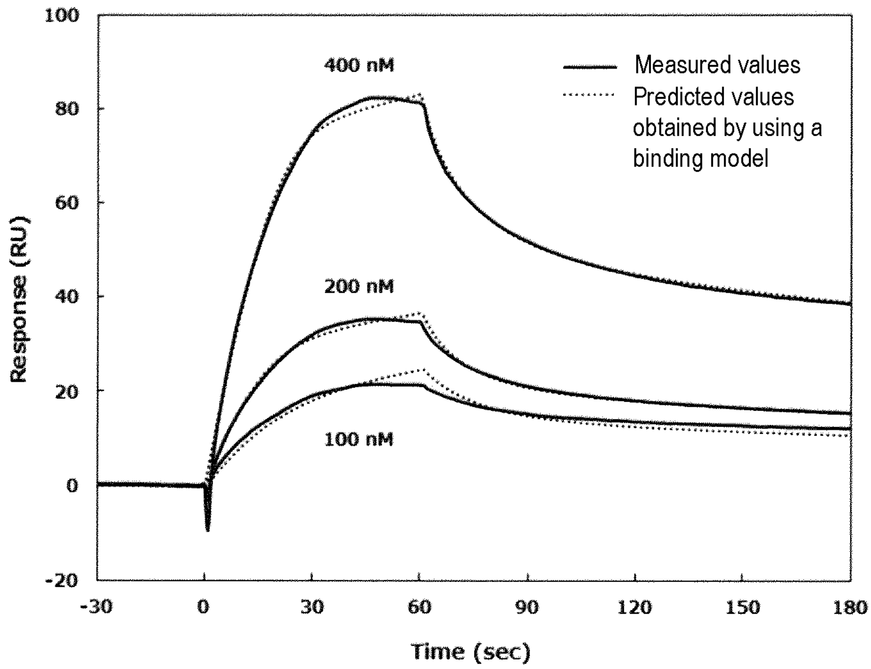
100
80
60
40
20
0
-20
Response (RU)
-30
0
30
60
90
120
150
180
Time (sec)
400 nM
200 nM
100 nM
Measured values
Predicted values obtained by using a binding model

Fig. 5

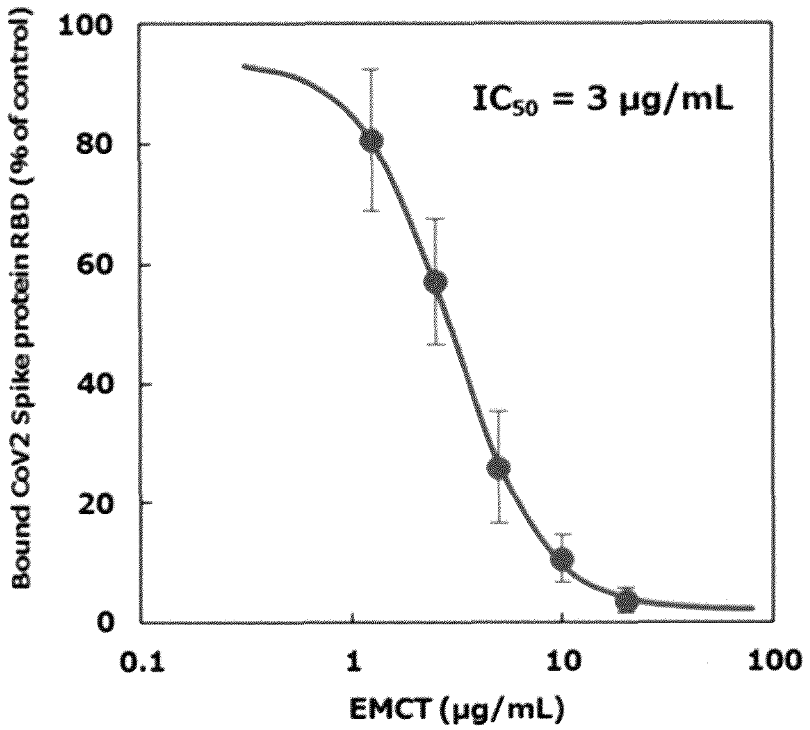


Fig. 6

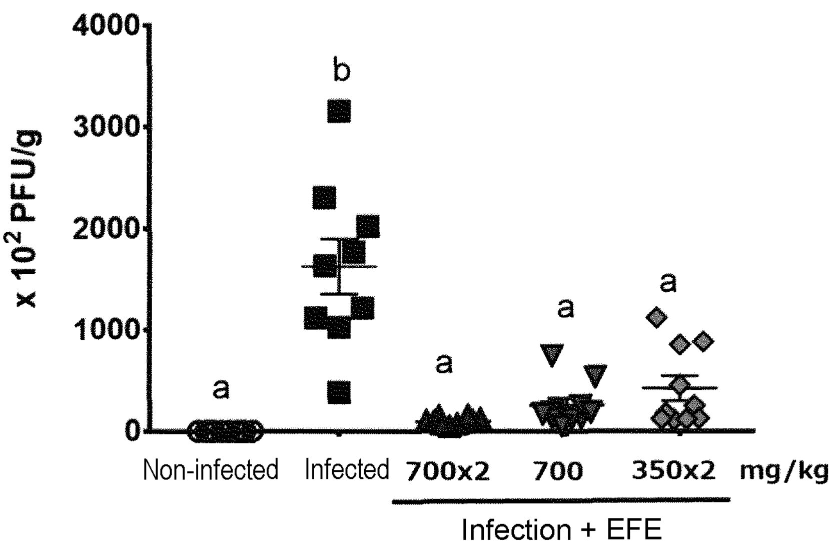


mean ± SE (non-infected group, each EFE group n = 10, infected group n = 9)
*P* < 0.01 by one-way ANOVA with Tukey's multiple comparison test

# Fig. 7

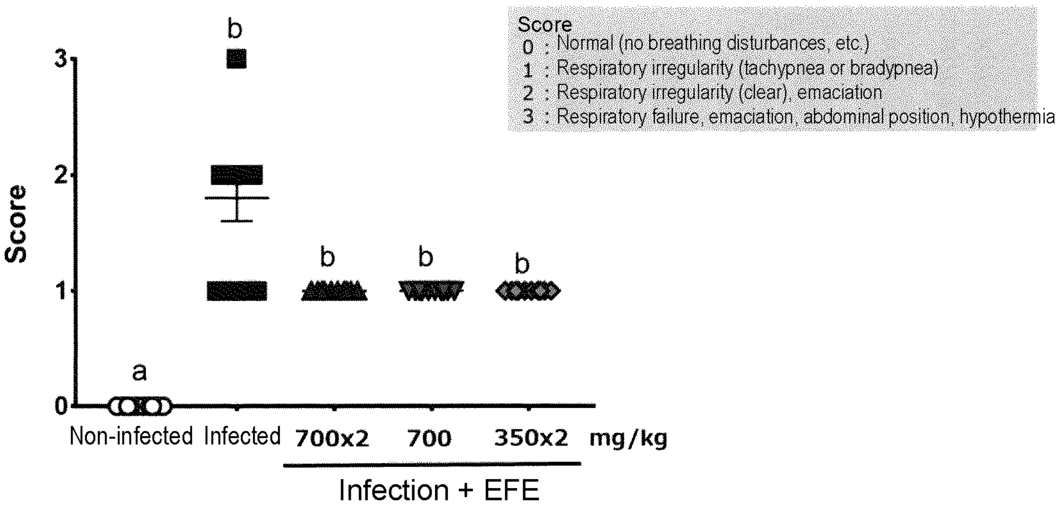


mean ± SE (n = 10 per group)
*P* < 0.01 by Kruskal-Wallis test with Dunn's multiple comparison test

# Fig. 8

Extension units

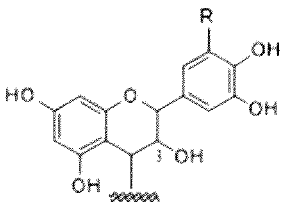

Terminal units

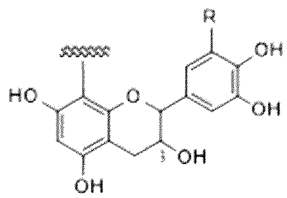

R=H: Catechol type
R=OH: Pyrogallol type

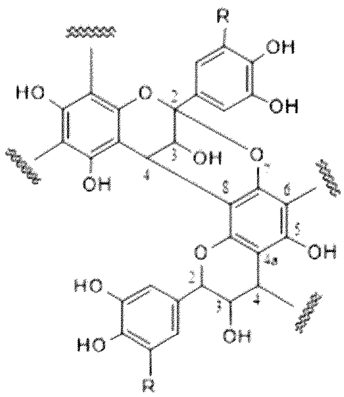

Proanthocyanidin A-type

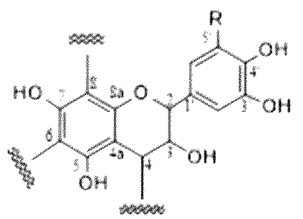

Proanthocyanidin B-type

## INTERNATIONAL SEARCH REPORT

International application No.
**PCT/JP2021/036413**

**A. CLASSIFICATION OF SUBJECT MATTER**

***A61K 36/17***(2006.01)i; ***A23L 33/105***(2016.01)i; ***A61P 31/14***(2006.01)i
FI: A61K36/17; A23L33/105; A61P31/14

According to International Patent Classification (IPC) or to both national classification and IPC

**B. FIELDS SEARCHED**

Minimum documentation searched (classification system followed by classification symbols)

A61K36/17; A23L33/105; A61P31/14

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | CN 111358892 A (XU, Xiu Xin) 03 July 2020 (2020-07-03)<br>abstract, example 4 | 1, 4-5, 7, 9, 11 |
| Y | abstract, example 4 | 1-11 |
| X | 小川恵子, 特別寄稿 COVID-19感染症に対する漢方治療の考え方(改訂 ver 2), 一般社団法人日本感染症学会ホームページ, お知らせ, April 2020, [retrieved on 22 October 2021], <https://www.kansensho.or.jp/uploads/files/news/gakkai/covid19_tokubetu2_0421.pdf> non-official translation (OGAWA, Keiko. Special Report. Chinese medicine therapy approaches to COVID-19 infection. (ver. 2), The Japanese Association for Infectious Diseases website. Notices.<br>p. 2/8, left column, paragraphs 1-4 | 1, 4-5, 7, 9, 11 |
| Y | p. 2/8, left column, paragraphs 1-4 | 1-11 |
| Y | WO 2015/076286 A1 (TOKIWA PHYTOCHEMICAL CO., LTD.) 28 May 2015 (2015-05-28)<br>claims 1, 10, examples 2, 10 | 2-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 October 2021** | **02 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/036413**

**C. DOCUMENTS CONSIDERED TO BE RELEVANT**

| Category* | Citation of document, with indication, where appropriate, of the relevant passages | Relevant to claim No. |
|---|---|---|
| Y | HYUGA, S. et al., Ephedrine alkaloids-free Ephedra Herb extract: a safer alternative to ephedra with comparable analgesic, anticancer, and anti-influenza activities, J. Nat. Med., 2016, vol. 70, pp. 571-583, ISSN: 1340-3443<br>abstract | 2-11 |
| Y | JP 2019-131536 A (KITASATO INST.) 08 August 2019 (2019-08-08)<br>claims 1, 8, examples 11-14 | 2-11 |
| Y | KR 10-0501831 B1 (KOREA RESEARCH INSTITUTE OF BIOSCIENCE) 20 July 2005 (2005-07-20)<br>examples, third line from the bottom in tables 1-3 | 2-11 |
| Y | YOSHIMURA, M. et al., Quality Evaluation and Characterization of Fractions with Biological Activity from Ephedra Herb Extract and Ephedrine Alkaloids-Free Epherda Herb Extract, Chem. Pharm. Bull., February 2020, vol. 68, pp. 140-149, ISSN: 1347-5223<br>abstract, p. 148, right column, third paragraph, fig. 3, table 1 | 2-11 |
| P, X | MEI, J. et al., Active components in Ephedra sinica stapf disrupt the interaction between ACE2 and SARS-CoV-2 RBD: Potent COVID-19 therapeutic agents, J. Ethnopharmacol., June 2021, Epub., vol. 278, #114303, ISSN: 0378-8741<br>abstract | 11 |
| P, X | LV, Y. et al., Screening and evaluation of anti-SARS-CoV-2 components from Ephedra sinica by ACE2/CMC-HPLC-IT-TOF-MS approach, Analytical & Bioanalytical Chemistry, February 2021, Epub., vol. 413, no. 11, pp. 2995-3004, ISSN: 1618-2642<br>abstract | 11 |

Form PCT/ISA/210 (second sheet) (January 2015)

**INTERNATIONAL SEARCH REPORT**
**Information on patent family members**

International application No.
**PCT/JP2021/036413**

| Patent document cited in search report | Publication date (day/month/year) | Patent family member(s) | Publication date (day/month/year) |
|---|---|---|---|
| CN 111358892 A | 03 July 2020 | (Family: none) | |
| WO 2015/076286 A1 | 28 May 2015 | CN 105744941 A<br>claims 1, 10, examples 2, 10<br>KR 10-2016-0086920 A | |
| JP 2019-131536 A | 08 August 2019 | CN 110090235 A<br>claims 1, 8, examples 11-14<br>KR 10-2019-0092314 A | |
| KR 10-0501831 B1 | 20 July 2005 | KR 10-2004-0097445 A<br>examples, third line from the bottom in tables 1-3 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015076286 A
- JP 2015549167 A
- JP 2019131536 A
- WO 2019131536 A


### Non-patent literature cited in the description


- Guide to Medical Treatment of New Coronavirus Infections, Version 5.3. Nikkan Yakugyo, 29 August 2021
- *Guide to the Medical Treatment of New Coronavirus Infections, Version 5.3,* 29 August 2021
- **WATANABE KENJI ; JIN CHENGIUN ; SHIBAYAMA CHIKANO ; LIU JIANPING ; JIA LIQUN ; JIN ; YAN HONGRONG.** Urgent Contribution] The Role of Kampo in New Coronavirus Infection (COVID-19). *Japan Medical Journal,* 18 April 2020, (5008), 44
- Special Contribution) How to Consider Kampo Therapy for COVID-19 Infections. **KEIKO OGAWA.** The Japanese Association for Infectious Diseases Website
- **RYUTARO ARITA ; MAKOTO TAKAYAMA ; KOTA ISHIZAWA ; TADASHI ISHII.** Report of Qingfei Paidu decoction for COVID-19 in China. *The Japanese Association for Infectious Diseases Website, http://www.kansensho.or.jp/modules/news/index.php?content_id=147*
- **OSHIMA N ; YAMASHITA T ; HYUGA S ; HYUGA M ; KAMAKURA H ; YOSHIMURA M ; MARUYAMA T ; HAKAMATSUKA T ; AMAKURA Y ; HANAWA T.** Efficiently prepared ephedrine alkaloids-free Ephedra Herb extract: a putative marker and antiproliferative effects. *J Nat Med,* 2016, vol. 70, 554-62
- **YOSHIMURA M ; AMAKURA Y ; HYUGA S ; HYUGA M ; NAKAMORI S ; MARUYAMA T ; OSHIMA N ; UCHIYAMA N ; YANG J ; OKA H.** Quality Evaluation and Characterization of Fractions with Biological Activities in Ephedra Herb Extract and Ephedrine Alkaloids-Free Ephedra Herb Extract. *Chem Pharm Bull,* 2020, vol. 68 (2), 140-149
- **ICHIRO NARIKAWA.** Kampo No Shucho (Opinion of Kampo)-Modern Sciences and Kampo Preparations. Kenyukan, Inc, 1991, 162-163
- **YOSHIMURA M ; AMAKURA Y ; HYUGA S ; HYUGA M ; NAKAMORI S ; MARUYAMA T ; OSHIMA N ; UCHIYAMA N ; YANG J ; OKA H.** Quality Evaluation and Characterization of Fractions with Biological Activities in Ephedra Herb Extract and Ephedrine Alkaloids-Free Ephedra Herb Extract. *Chem Pharm Bull.,* 2020, vol. 68 (2), 140-149
- **ALBUQUERQUE, N et al.** *J Virol,* 2006, vol. 80, 10382-10394
- Basic Guidelines for Conduct of Animal Experiments at Research Institutes. Ministry of Health, Labour and Welfare